# EUROPEAN PATENT APPLICATION

(11) **EP 3 950 040 A2**
(43) Date of publication of application: **09.02.2022**
(21) Application number: 21188151.1
(22) Date of filing: 28.07.2021
(51) Int. Cl.: A61M 25/10, B29C 65/00, A61M 25/00

(54) **BALLOON GUIDE CATHETER HAVING REDUCED OUTER DIAMETER DISTAL AND PROXIMAL BONDING INTERFACE AREAS WITH THE BALLOON**

(30) Priority: 29.07.2020 US 202016942684
(71) Applicant: Neuravi Limited, Galway H91 K5YD (IE)
(72) Inventor: CONNOLLY, Patrick, Galway, H91 K5YD (IE); CONWAY, Shane, Galway, H91 K5YD (IE); CONLON, Richard, Galway, H91 K5YD (IE)
(74) Representative: Carpmaels & Ransford LLP

(57) **Abstract**

A balloon guide catheter (100) including a balloon (145) and a catheter shaft (105) having a central lumen (107) defined axially therethrough, and an inflation lumen (135). An exterior surface of the catheter shaft has a distal reduced outer diameter recess area (120A) defined therein and a proximal reduced outer diameter recess area (120B) separated in an axial direction from the distal reduced outer diameter recess area with a discharge port (140) of the inflation lumen disposed therebetween. The balloon is secured to the catheter shaft by adhesive pooled in the distal reduced outer diameter recess area forming a distal bonding interface area; and the proximal reduced outer diameter recess area forming a proximal bonding interface area. Heat shrink tubing is positioned about the balloon at the distal and proximal bonding interface areas.

## Description

### BACKGROUND OF THE INVENTION

### Field of the Invention

An intravascular catheter and, in particular, a balloon guide catheter during capture and retrieval of a thrombus, occlusion, or clot by impeding blood flow through a vessel. The balloon and exterior surface of the catheter shaft of the present inventive balloon guide catheter are secured together at bonding interface areas having a minimized outer diameter and optimized bond strength.

### Description of Related Art

Acute ischemic stroke is primarily caused by a thrombotic or embolic occlusion (e.g., blockage) in an artery of the brain. The occlusion is typically caused by a blood clot liberated from another part of the body which travels in an antegrade direction (in the direction of normal blood flow) through the vessel and eventually becomes lodged in a neurovascular artery, where it obstructs blood flow to a region of the brain.

A procedure known as a thrombectomy may be used to remove the thrombus, occlusion, blockage or clot lodged in the vessel using a mechanical retrieval device. During the thrombectomy procedure or treatment a physician or interventionalist endovascularly introduces a guidewire and microcatheter together through the vasculature, typically in an artery located in the groin or the arm or by direct access through the carotid artery. Together the guidewire and microcatheter are advanced to a location facing a proximal side of the targeted clot, blockage or occlusion. Then the guidewire is advanced across the clot, followed by the microcatheter. While in a compressed state, a mechanical thrombectomy device may be guided through the lumen of the microcatheter to the target site. Upon emerging from the microcatheter the mechanical thrombectomy device typically automatically expands to its original enlarged state. Mechanical thrombectomy devices are typically made of a self-expanding biocompatible material such as nickel-titanium. Aspiration through the catheter may accompany or be used in place of the mechanical retrieval device to remove the clot.

During a thrombectomy procedure balloon guide catheters are often employed to arrest blood flow by introducing an inflation fluid into a compliant inflatable balloon (rather than inflating via pressure) made of an elastomeric material, for example, polyurethane, polyblend, or latex. Its ability to conform to the shape of the vasculature makes the compliant inflatable balloon particularly suited for use in arresting of blood flow. In other applications such as dilating of a vessel or opening an occlusion, balloon guide catheters may employ a non-compliant or semi-compliant balloon that is inflated by pressure, rather than using an inflation fluid. Specifically, non-compliant balloons typically made of polyester or nylon when inflated at a high pressure dilate a vessel or open an occlusion; whereas semi-compliant balloons made of material such as Pebax or higher durometer polyurethanes when inflated in pressure are more compliant than that of non-compliant balloons providing greater flexibility during delivery. Regardless of the type of balloon (compliant, semi-compliant, or non-compliant), bonding of the balloon to the exterior surface of the catheter shaft during manufacture has two competing criteria, i.e., minimization of the outer profile/diameter at the bonding interface area(s) in which the balloon is mounted to the catheter shaft while maximizing bond strength and integrity.

It is desirable to design an improved balloon guide catheter having a bonding interface area between the balloon and outer surface of the catheter shaft to achieve optimum bond strength without increasing the outer diameter.

### Summary of the Invention

An aspect of the present invention is directed to an improved balloon guide catheter having a bonding interface area between the balloon and outer surface of the catheter shaft with a minimum outer diameter and enhanced bonding strength.

Another aspect of the present invention is directed to a balloon guide catheter including a balloon and a catheter shaft having a central lumen defined axially therethrough, and an inflation lumen. An exterior surface of the catheter shaft has a distal reduced outer diameter recess area defined therein and a proximal reduced outer diameter recess area separated in an axial direction from the distal reduced outer diameter recess area with a discharge port of the inflation lumen disposed therebetween. The balloon is secured to the catheter shaft by adhesive pooled in the distal reduced outer diameter recess area forming a distal bonding interface area; and the proximal reduced outer diameter recess area forming a proximal bonding interface area. Heat shrink tubing is positioned about the balloon at the distal and proximal bonding interface areas.

Still another aspect of the present invention relates to a method for assembling a balloon guide catheter including a balloon having a distal edge and an opposite proximal edge; and a catheter shaft having an exterior surface, a central lumen defined axially therethrough, and an inflation lumen. The exterior surface has a distal reduced outer diameter recess area defined therein and a proximal reduced outer diameter recess area separated in an axial direction from the distal reduced outer diameter recess area. The inflation lumen having a discharge port disposed between the distal and proximal reduced outer diameter recess areas. The balloon is secured within: (i) the distal reduced outer diameter recess area forming a distal bonding interface area; and (ii) the proximal reduced outer diameter recess area forming a proximal bonding interface area. The method of assembly including securing the balloon to the catheter shaft using biocompatible adhesive pooled in the distal and proximal reduced outer diameter recess areas. Thereafter, laser or thermal bonding is applied to heat shrink tubing disposed about the distal and proximal bonding interface areas of the secured compliant balloon fusing the compliant balloon to the catheter shaft.

### Brief Description of the Drawing

The foregoing and other features of the present invention will be more readily apparent from the following detailed description and drawings illustrative of the invention wherein like reference numbers refer to similar elements throughout the several views and in which:
Figure 1A is a partial distal end perspective view of an embodiment of the catheter shaft of the present inventive balloon guide catheter prior to securing a compliant balloon about the exterior surface thereof; wherein an exterior surface of the catheter shaft has distal and proximal reduced outer diameter recess areas (e.g., respective 360° radial grooves) defined therein;
Figure 1B is a partial axial cross-sectional view of an assembled balloon guide catheter with the compliant balloon secured using heat shrink tubing and a biocompatible adhesive pooled within the distal and proximal reduced outer diameter recess areas (e.g., 360° radial grooves) defined in the exterior surface of the catheter shaft of Figure 1A, wherein the compliant balloon is shown in an inflated state;
Figure 1C is a radial cross-sectional view through the distal reduced outer diameter recess area (e.g., 360° radial groove) defined in the exterior surface of the catheter shaft along lines I(C)-I(C) in Figure 1B;
Figure 1D is a radial cross-sectional view through the proximal reduced outer diameter recess area (e.g., 360° radial groove) defined in the exterior surface of the catheter shaft along lines I(D)-I(D) in Figure 1B;
Figure IE is a perspective cut-out illustration of a partial section of the catheter shaft of Figure 1A depicting the braid woven above and below the inflation lumen;
Figure 2A is a distal end perspective view of another embodiment of the catheter shaft of the present inventive balloon guide catheter prior to securing a compliant balloon about the exterior surface thereof; wherein an exterior surface of the catheter body has a distal reduced outer diameter recess area (e.g., 360° radial groove) and a proximal reduced outer diameter recess area (e.g., radial groove less than 360°) defined therein;
Figure 2B is a side view of the catheter shaft of Figure 2A;
Figure 2C is a radial cross-sectional view through the distal reduced outer diameter recess area (e.g., 360° radial groove) defined in the exterior surface of the catheter shaft along lines II(C)-II(C) in Figure 2A;
Figure 2D is a radial cross-sectional view through the proximal reduced outer diameter recess area (e.g., radial groove less than 360°) defined in the exterior surface of the catheter shaft along lines II(D)-II(D) in Figure 2A;
Figure 3A is a distal end perspective view of another embodiment of the catheter shaft of the present inventive balloon guide catheter prior to securing a compliant balloon about the exterior surface thereof; wherein an exterior surface of the catheter body has distal and proximal reduced outer diameter recess areas (e.g., longitudinal channels) defined therein;
Figure 3B is a radial cross-sectional view through the distal reduced outer diameter recess area (e.g., longitudinal channels) defined in the exterior surface of the catheter shaft along lines III(B)-III(B) in Figure 3A;
Figure 3C is a radial cross-sectional view through the proximal reduced outer diameter recess area (e.g., longitudinal channels) defined in the exterior surface of the catheter shaft along lines III(C)-III(C) in Figure 3A;
Figure 3D is a distal perspective view of the assembled balloon guide catheter with a compliant balloon secured using heat shrink tubing and a biocompatible adhesive pooled within the distal and proximal reduced outer diameter recess areas (e.g., longitudinal channels) defined in the exterior surface of the catheter shaft of Figure 3A, wherein the compliant balloon is shown in a non-inflated state;
Figure 4A is a distal end perspective view of yet another embodiment of the catheter shaft of the present inventive balloon guide catheter prior to securing a compliant balloon about the exterior surface thereof; wherein an exterior surface of the catheter body has distal and proximal reduced outer diameter recess areas (e.g., a series of radially arranged wells) defined therein;
Figure 4B is a distal end perspective view of the catheter shaft of Figure 4A with the non-inflated compliant balloon positioned about the exterior surface thereof; wherein distal and proximal edges of the deflated compliant balloon are rolled towards one another exposing the distal and proximal reduced outer diameter recess areas (e.g., a series of radially arranged wells) defined in the exterior surface of the catheter shaft;
Figure 4C is a radial cross-sectional view through the distal reduced outer diameter recess area (e.g., series of radially arranged wells) defined in the exterior surface of the catheter shaft along lines IV(C)-IV(C) in Figure 4A; and
Figure 4D is a radial cross-sectional view through the proximal reduced outer diameter recess area (e.g., series of radially arranged wells) defined in the exterior surface of the catheter shaft along lines IV(D)-IV(D) in Figure 4A.

### Detailed Description of the Invention

The terms "distal" or "proximal" are used in the following description with respect to a position or direction relative to the treating physician or medical interventionalist. "Distal" or "distally" are a position distant from or in a direction away from the physician or interventionalist. "Proximal" or "proximally" or "proximate" are a position near or in a direction toward the physician or medical interventionalist. The terms "occlusion", "clot" or "blockage" are used interchangeably.

By way of example, the present inventive balloon guide catheter has been illustrated and described using a compliant inflatable balloon for arresting blood flow through the vessel. Any type of balloon (e.g., compliant, semi-compliant, non-compliant) may be used with the present inventive catheter to arrest blood flow or enlarge the vessel. The balloon guide catheter includes a balloon secured at distal and proximal bonding interface areas about an exterior surface of a catheter shaft, wherein the distal bonding interface area is located on the catheter shaft distally of and separated in an axial direction from the proximal bonding interface area. In alignment with the distal and proximal bonding interface areas, the exterior surface of the catheter shaft has respective recesses defined therein reduced in outer diameter relative to the outer diameter of the catheter shaft in remaining non-bonding interface areas. A distal reduced outer diameter recess area having one or more recesses defined in the exterior surface of the catheter shaft is aligned with the distal bonding interface area, while a proximal reduced outer diameter recess area having one or more recesses defined in the exterior surface of the catheter shaft is aligned with the proximal bonding interface area. Distal and proximal reduced outer diameter recess areas are distinct and separate from one another in an axial direction. Several non-limiting exemplary configurations of the distal and proximal reduced outer diameter recess areas defined in the exterior surface of the catheter shaft are discussed in detail below but other configurations are contemplated and within the scope of the present invention. A biocompatible adhesive is injected into the distal and proximal reduced outer diameter recess areas. Post gluing, the balloon is positioned over the adhesive and subjected to thermal bonding or laser bonding to optimize the bond therebetween.

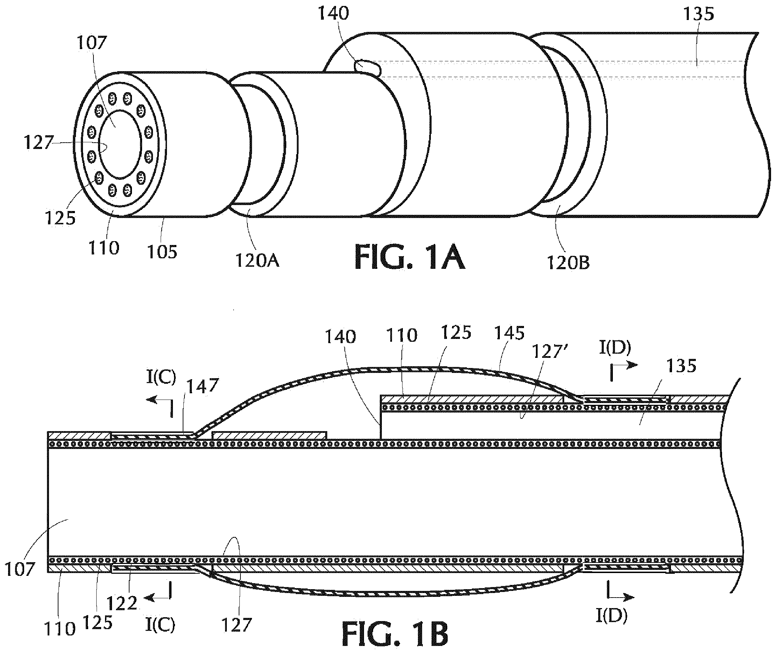

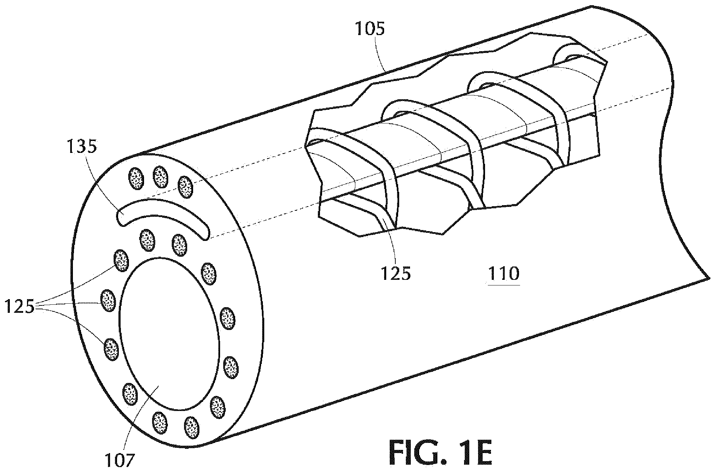

Figure 1A is a distal end perspective view of a catheter shaft 105 of the present inventive balloon guide catheter. Preferably, catheter shaft 105 comprises an outer layer 110 and a supporting layer 125 disposed radially inward thereof, as shown in Figures 1B-1D. Supporting layer 125 is any type of mechanical supporting structure providing enhanced strength, flexibility, and kink resistance to the catheter. The supporting layer 125 is depicted in the figures as a braid, but other mechanical structures may be employed such as a coil, a mesh, a hypotube, or a polymer component. An inner liner or lining 127 forming a central lumen 107 in the catheter shaft is disposed radially inward and in direct physical contact with the supporting layer 125. Distal and proximal outer diameter recess areas in the configuration shown in Figure 1A are configured as a single distal and a single proximal radial groove or channel 120A, 120B, respectively. Each of the distal and proximal radially defined grooves or channels 120A, 120B, preferably, has a radial depth or radial thickness constrained exclusively to the outer layer 110 (i.e., not extending radially inwardly into the underlying supporting layer 125). More preferably, the radial depth/thickness of each of the distal and proximal radial grooves or channels 120A, 120B is substantially equal to the radial depth/thickness of the outer layer 110 thereby exposing an exterior surface of the supporting layer 125 to which the adhesive physically contacts forming a stronger bond. Inner liner 127 remains intact preventing leakage of the adhesive radially inward beyond the supporting layer 125 into the central lumen 107.

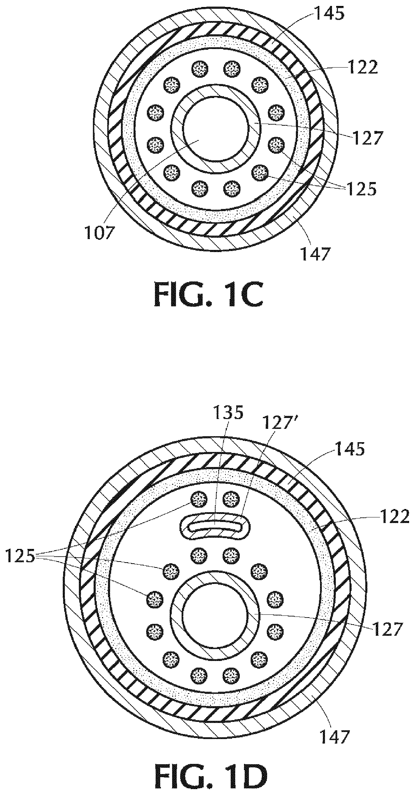

Parallel to the central lumen 107 is an inflation lumen 135 extending axially within a portion of the outer layer 110 of the catheter shaft 105 terminating at its distal end with a discharge port 140 disposed between the distal and proximal radial grooves or channels 120A, 120B, as shown in Figures 1A & 1B. Similar to the central lumen being defined by the inner layer or lining 127, the inflation lumen 135 is also defined by an inner layer or lining 127'. The inner layer or lining 127, 127' may, but need not necessarily be, the same material. Preferably, both the central lumen 107 and the inflation lumen 135 are supported by the braid 125 (e.g., supporting layer). That is, the braid 125 encompasses or surrounds not only the central lumen 107, but is also woven above and below the inflation lumen 135 (similar to a figure "8"), as illustrated in the cut-out perspective view of the catheter shaft in Figure IE. As is shown in the radial cross-sectional view of Figure 1C, a single distal reduced outer diameter recess area (e.g., radial groove) 120A preferably extends 360° about the entire circumference of the exterior surface of the catheter shaft 105. A single radial recess or groove extending radially less than 360° about the circumference of the exterior surface of the catheter shaft is also possible at the sacrifice of a reduction in overall bond strength.

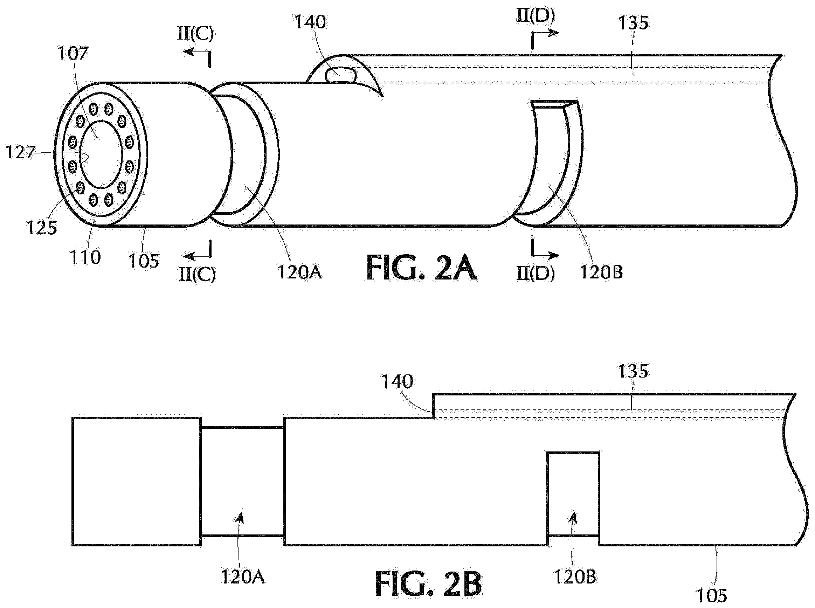

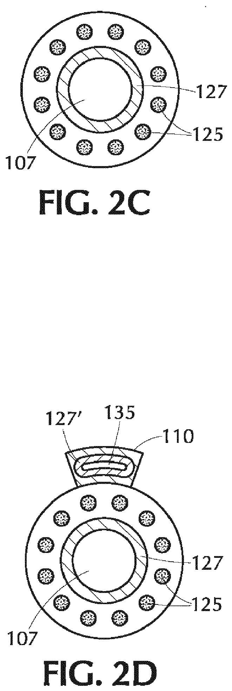

A single proximal reduced outer diameter recess area (e.g., radial groove) 120B extends preferably 360° about the circumference of the exterior surface of the catheter shaft encircling both the central lumen 107 and the inflation lumen 135. Despite the radial groove 120B encompassing the inflation lumen 135, sufficient support or strength is imparted by the braid 125 woven above and below the inflation lumen to prevent kink resistance, as shown in Figure IE. However, if the catheter is configured so that the inflation lumen 135 is not supported by the braid 125 (e.g., the braid is not woven above and below the inflation lumen, instead disposed only about the central lumen) then the proximal reduced outer diameter recess area (e.g., radial groove) 120b preferably extends less than 360° about the circumference of the exterior surface of the catheter shaft so that a non-reduced outer diameter radial section (e.g., arc) of the outer layer 110 of the catheter shaft 105 maintains a non-reduced outer diameter without the proximal radial groove or channel 120B defined radially inward therein, as illustrated in Figures 2A-2D. Referring to Figure 2D representing the radial cross-sectional view through the proximal reduced outer diameter radial section of the catheter shaft along line II(D) ― II(D) in Figure 2A, the non-reduced outer diameter radial section (arc) of the exterior surface of the catheter shaft is substantially aligned with and sufficient in radius to encompass therein the inflation lumen 135. In other words, the extent of the proximal reduced outer diameter recess area (e.g., radial groove) 120B about the circumference of the exterior surface of the catheter shaft in the embodiment depicted in Figures 2A-2D is constrained, limited or restricted so as not to disrupt, interfere, or intersect with the inflation lumen 135 when not supported by the braid 125. The strength of the bond will be diminished or weakened along the non-reduced outer diameter radial section (e.g., arc) of the outer layer relative to the bond strength within the reduced-outer diameter radial section.

Only a single radial groove 120A, 120B is shown and described in the embodiment of Figure 1A for each of the distal and proximal reduced outer diameter recess areas. However, more than one radial groove may be defined in each of the distal and proximal reduced outer diameter recess areas. When more than one radial groove is defined in each reduced outer diameter recess area (e.g., distal and proximal reduced outer diameter recess areas), each radial groove may be sized and arranged as desired. For example, the radial grooves within each reduced outer diameter recess area may be: (i) parallel or non-parallel to one another; (ii) the same or differ in width in an axial direction.

Referring to the assembled balloon guide catheter 100 in Figure 1B, biocompatible adhesive 122 is injected into the distal and proximal reduced outer diameter recess areas (e.g., radial grooves) 120A, 120B pooling therein to secure distal and proximal bonding interface areas of the compliant balloon 145 to the exterior surface of the catheter shaft 105. Figure 1C is a radial cross-sectional view along lines I(C)-I(C) through the distal radial groove or channel 120A of the assembled balloon guide catheter in Figure 1B. Each of the central lumen 107 and the inflation lumen parallel thereto is defined by an inner lining or liner 127, 127', respectively. Braid 125 is disposed about the inner liner 127 forming the central lumen 107 as well as being woven above and below the inner liner 127' forming the inflation lumen 135, as depicted in Figure 1D representative of a radial cross-sectional view through the proximal reduced outer diameter recess along lines 1(D)-1(D) of the assembled balloon guide catheter in Figure 1B. The biocompatible adhesive 122 pooled within the distal reduced outer diameter recess 120A secures the balloon 145 to the exposed supporting layer 125 (e.g., braid) of the catheter shaft. As a result of the pooling of the adhesive or glue 122 in the reduced outer diameter radial grooves 120A, 120B the outer diameter of the assembled balloon guide catheter (at the distal and proximal bonding interface areas) is minimized while maximizing bond strength between the compliant balloon and the catheter shaft. To further enhance bond strength between the balloon and catheter shaft, heat shrink tubing 147 disposed about the balloon at the distal and proximal bonding interface areas is laser bonded or thermally bonded fusing the balloon to the exterior surface of the catheter.

At the risk of a weakened bond between the balloon and exterior surface of the catheter shaft, it is possible to eliminate the use of an adhesive or glue. Distal and proximal bonding interface areas of the compliant balloon 145 may instead be fused to the catheter shaft using only heat shrink tubing aligned with the distal and proximal reduced outer diameter recess areas (e.g., radial grooves) 120A, 120B, respectively.

Adhesive bonding (e.g., glue), thermal bonding (e.g., hot jaw bonders applied about the heat shrinkable tubing causing the balloon and the catheter shaft to flow melt together), laser bonding and/or mechanical bonding (e.g., crimped band) may be utilized to secure the distal and proximal bonding interface areas of the compliant balloon 145 to the distal and proximal reduced outer diameter recess areas (e.g., radial grooves) 120A, 120B. These enumerated bonding methods apply to all embodiments, configurations and designs illustrated and described herein.

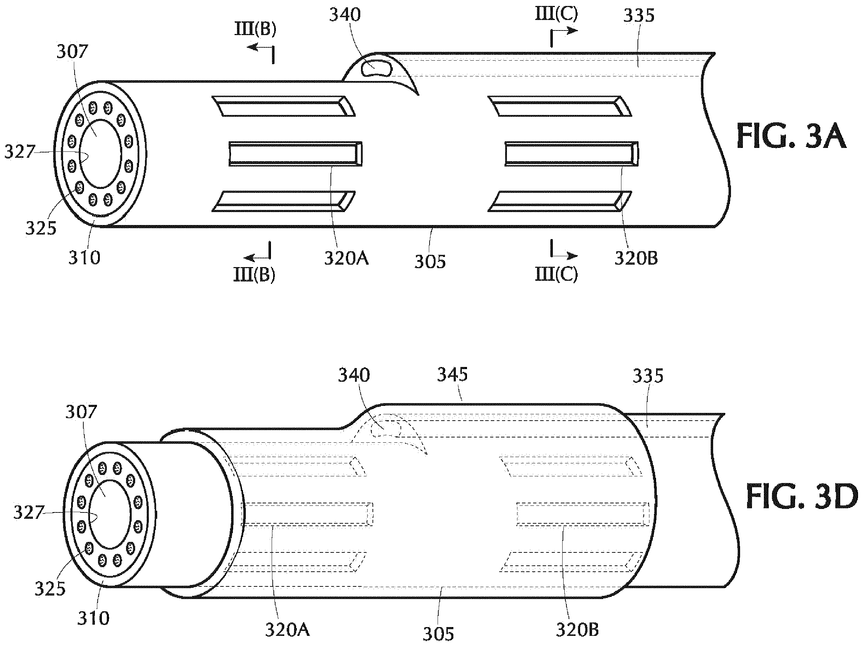

In yet another configuration shown in Figures 3A-3D the distal and proximal reduced outer diameter recess areas defined in the exterior surface of the catheter shaft are longitudinal channels 320A, 320B arranged substantially parallel with one another in an axial direction. Referring to Figure 3A, a distal reduced outer diameter recess area includes a first set of plural axial/longitudinal channels 320A defined in the exterior surface of the catheter shaft 305. Distinct and separate in an axial direction from the first set, the proximal reduced outer diameter recess area includes a second set of plural axial/longitudinal channels 320B defined in the exterior surface of the catheter shaft 305.

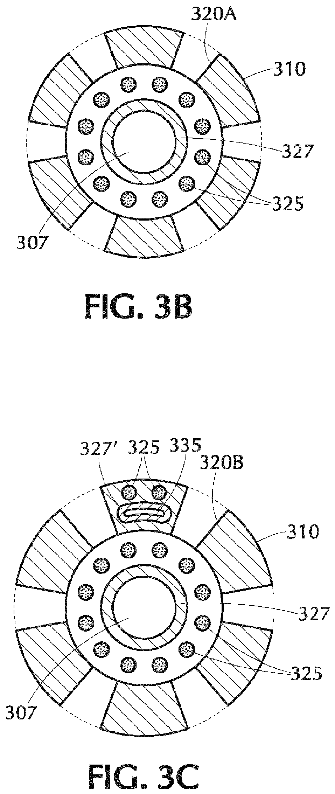

By way of example, six longitudinal channels 320A are defined in the exterior surface of the catheter shaft for the distal reduced outer diameter recess area, as depicted in the radial cross-sectional view in Figure 3B. A radial cross-sectional view through the proximal reduced outer diameter recess area (e.g., longitudinal channels) 320B is shown in Figure 3C showing that the longitudinal channels comprising the proximal reduced outer diameter recess area defined in the exterior surface of the outer layer do not interfere with (avoid) the inflation lumen 335. The plural longitudinal channels 320A, 320B are preferably uniform in axial length (preferably, ≤ approximately 1mil = 0.001 inch). For each of the distal and proximal reduced outer diameter recess areas any one or more variations of the longitudinal channels may be selected, as desired: (i) the number of longitudinal channels 320A, 320B may be varied; (ii) in a lateral direction, the spacing between adjacent longitudinal channels; (iii) in a lateral direction the width of each of the plural longitudinal channel; and (iv) the radial depth/radial thickness of each longitudinal channel in the exterior surface of the catheter shaft is less than or equal to that of the outer layer (most preferably, equal to that of the outer layer thereby exposing the supporting layer below). The discharge port 340 of the inflation lumen 335 is disposed between the distal and proximal reduced outer diameter recess areas (e.g., longitudinal grooves) 320A, 320B. Like the figure "8" configuration in Figures 1A-1D described above, braid 325 is preferably woven above and below the inflation lumen 335 in the catheter shaft configuration in Figures 3A-3D. However, the configuration shown in Figures 3A-3D may be modified in accordance with the present invention wherein the inflation lumen is not supported by the braid 325.

Referring to Figure 3D, while in a non-inflated state the compliant balloon 345 is positioned about the exterior surface of the catheter shaft 305. The distal and proximal edges of the compliant balloon 345 are rolled towards one another exposing the distal and proximal reduced outer diameter longitudinal channels 320A, 320B. Biocompatible adhesive (e.g., glue) is injected into the exposed distal and proximal reduced outer diameter longitudinal channels 320A, 320B. Thereafter, the rolled distal and proximal edges of the compliant balloon 345 are unfurled covering the distal and proximal reduced outer diameter recess areas (e.g., longitudinal channels) 320A, 320B with the pooled adhesive therein forming the distal and proximal bonding interface areas. As with the configurations described heretofore, heat shrink tubing positioned at the distal and proximal bonding interface areas is subject to thermal bonding or laser bonding to reflow melt the compliant balloon to the catheter shaft. Any combination of adhesive, thermal, laser and/or mechanical bonding processes may be employed for securing the compliant balloon to the catheter shaft.

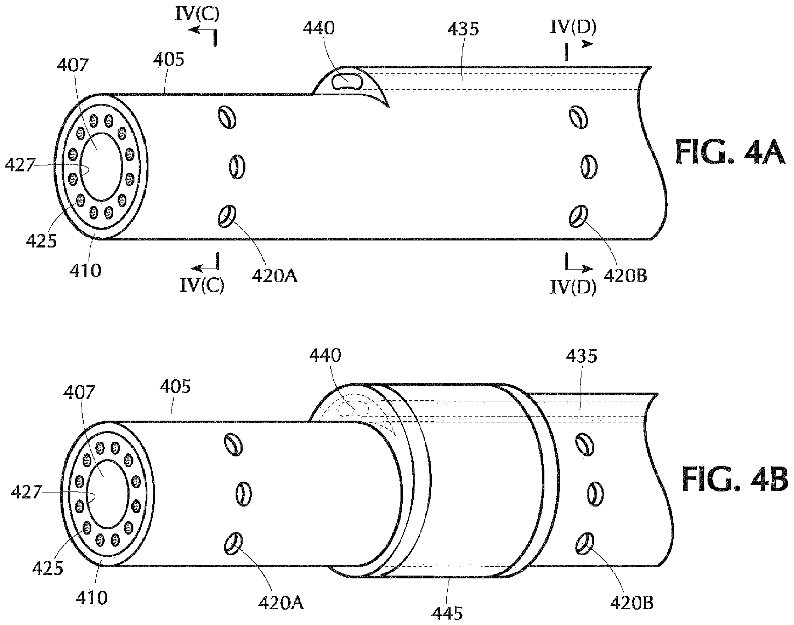

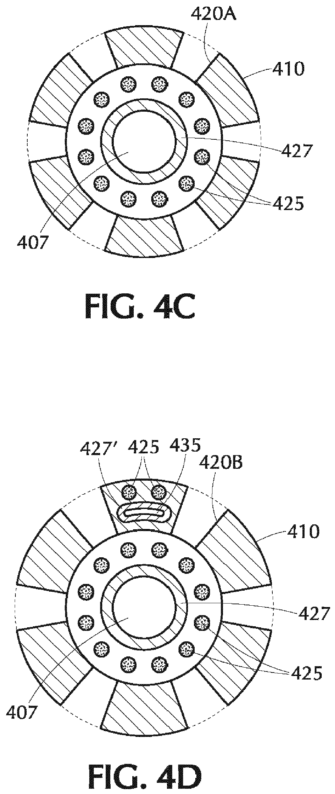

Figures 4A-4D illustrate still another configuration of the distal and proximal reduced outer diameter recess areas defined in the exterior surface of the catheter shaft as a radial row or series of wells or perforations 420A, 420B extending 360° at the same axial distance along the catheter shaft 405. Wells 420B comprising the proximal reduced outer diameter recess area are arranged so as not to interfere (avoid) the inflation lumen 435 extending axially through the outer layer 410 of the catheter shaft 405, as shown in Figure 4D. The shape of each well, dimension of each well, spacing between adjacent wells, and/or number of the wells may be constant or varied, as desired. As with the previously described configurations, the radial depth or radial thickness of each well is preferably less than or equal to the radial depth or radial thickness of the outer layer 410, most preferably equal to that of the outer layer 410 exposing the braid 425 (e.g., supporting layer) beneath. The radial depth or radial thickness of each well need not be constant. Each well may be any desired shape. By way of illustration only, circular shape wells each approximately 1 mil = 0.001 inch are defined in the exterior surface of the catheter shaft 405 represented in Figures 4A ― 4D. Other geometric shapes of any desired dimension are possible such as square, rectangular (i.e., radial sections or arcs), oval, or triangular. Wells may be drilled in the outer surface of the catheter shaft using such techniques as drilling or hot burning. Only a single row (single band) of radially defined wells is illustrated for each of the distal and proximal reduced outer diameter recess areas 420A, 420B in Figures 4A-4D. More than one row (e.g., band) of radially defined wells may be provided axially offset from one another, each well in adjacent rows (e.g., bands) may be aligned or non-aligned (offset) radially with one another.

Numerous techniques may be used to define the distal and proximal reduced outer diameter recess areas (e.g., radial grooves, longitudinal channels, or wells) in the exterior surface of the catheter shaft. Drilling into the exterior surface (e.g., outer layer) is one technique for creating the desired reduced outer diameter recesses in the exterior surface of the catheter. Other conventional methods include thermal burning the outer layer of the exterior surface of the catheter shaft to create the desired reduced outer diameter recess areas.

During assembly the compliant balloon while in a non-inflated (e.g., deflated) state is positioned about the exterior surface of the catheter shaft covering the distal and proximal reduced outer surface recess areas (e.g., distal and proximal series of radially arranged wells). The distal and proximal edges of the balloon are rolled back onto themselves towards one another exposing the distal and proximal reduced outer diameter recess areas, as depicted in Figure 4B. The biocompatible adhesive or glue is injected into the exposed distal and proximal reduced outer diameter recess areas. Thereafter, the rolled distal and proximal edges of the compliant balloon are unfurled (rolled back exposing their respective distal and proximal edges) over the distal and proximal reduced outer diameter recess areas with the adhesive pooled therein forming secure distal and proximal bonding interface areas therebetween. Heat shrink wrap tubing is then positioned about the distal and proximal bonding interface areas. Post gluing, the bonds undergo thermal bonding or laser bonding causing the reflow melting or fusing of the balloon to the exterior surface of the catheter shaft. The biocompatible adhesive or glue is advantageous because polyblend balloons don't sufficiently reflow or melt when thermally bonded or laser bonded resulting in an inadequate weak bond. Thermal bonding or laser bonding processing performed post gluing tacks down any portion of the balloon material not glued to the exterior surface of the catheter shaft as well as eliminates distal and proximal edges of the balloon that are reflow melted onto the catheter shaft.

If mechanical and/or thermal bonding processes are substituted for adhesive bonding (i.e., adhesive bonding is eliminated altogether) to secure the compliant balloon within the distal and proximal reduced outer diameter recess areas the need for rolling up/unfurling the balloon to inject the adhesive is eliminated. The compliant balloon while in a non-inflated (e.g., deflated) state is positioned about the exterior surface of the catheter shaft covering the distal and proximal reduced outer surface recess areas. Thereafter, mechanical and/or thermal bonding processes are instituted in a region of the compliant balloon disposed within the distal and proximal reduced outer diameter recess areas forming secure bonds distal and proximal bond interface areas therebetween.

Thus, while there have been shown, described, and pointed out fundamental novel features of the invention as applied to a preferred embodiment thereof, it will be understood that various omissions, substitutions, and changes in the form and details of the systems/devices illustrated, and in their operation, may be made by those skilled in the art without departing from the spirit and scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

Every issued patent, pending patent application, publication, journal article, book or any other reference cited herein is each incorporated by reference in their entirety.

## Claims

1. A balloon guide catheter comprising:
a balloon having a distal edge and an opposite proximal edge; and
a catheter shaft having an exterior surface, a central lumen defined axially therethrough, and an inflation lumen; wherein the exterior surface of the catheter shaft has a distal reduced outer diameter recess area defined therein and a proximal reduced outer diameter recess area separated in an axial direction from the distal reduced outer diameter recess area; the inflation lumen having a discharge port disposed between the distal and proximal reduced outer diameter recess areas;
wherein the balloon is secured at: (i) the distal reduced outer diameter recess area forming a distal bonding interface area; and (ii) the proximal reduced outer diameter recess area forming a proximal bonding interface area.

2. The balloon guide catheter of claim 1, wherein the distal reduced outer diameter recess area is a first set of a plurality of longitudinally defined channels defined in the exterior surface of the catheter shaft separated in an axial direction from so as to be distinct from the proximal reduced outer diameter recess area having a second set of a plurality of longitudinally defined channels defined in the exterior surface of the catheter shaft.

3. The balloon guide catheter of claim 1, wherein the distal reduced outer diameter recess area is a first set of at least one radial row of wells defined in the exterior surface of the catheter shaft separated in an axial direction distinct from the proximal reduced outer diameter recess area having a second set of at least one radial row of wells defined in the exterior surface of the catheter shaft.

4. The balloon guide catheter of claim 1, further comprising a biocompatible adhesive pooled within the distal and proximal reduced outer diameter recess areas.

5. The balloon guide catheter of claim 1, further comprising heat shrink tubing positioned about the balloon at the distal and proximal bonding interface areas.

6. A method for assembling a balloon guide catheter including a balloon having a distal edge and an opposite proximal edge; and a catheter shaft having an exterior surface, a central lumen defined axially therethrough, and an inflation lumen;
wherein the exterior surface has a distal reduced outer diameter recess area defined therein and a proximal reduced outer diameter recess area separated in an axial direction from the distal reduced outer diameter recess area; the inflation lumen having a discharge port disposed between the distal and proximal reduced outer diameter recess areas; and the balloon is secured within: (i) the distal reduced outer diameter recess area forming a distal bonding interface area; and (ii) the proximal reduced outer diameter recess area forming a proximal bonding interface area; the method comprising the steps of:
securing the balloon to the catheter shaft using biocompatible adhesive pooled in the distal and proximal reduced outer diameter recess areas; and
applying laser or thermal bonding to heat shrink tubing disposed about the distal and proximal bonding interface areas of the secured balloon fusing the balloon to the catheter shaft.

7. The balloon guide catheter of claim 1 or the method of claim 6, wherein the catheter shaft includes:
an outer layer,
a supporting layer disposed radially inward and in direct contact with the outer layer; and
an inner liner disposed radially inward and in direct contact with the supporting layer;
wherein the distal and proximal reduced outer diameter recess areas have a radially inward depth less than or equal to that of the outer layer.

8. The method of claim 6, wherein the securing step comprises:
positioning the balloon about the exterior surface of the catheter shaft at least partially covering the distal and proximal reduced outer diameter recess areas;
rolling the distal and proximal edges of the balloon towards one another exposing the distal and proximal reduced outer diameter recess areas;
pooling the biocompatible adhesive in the distal and proximal reduced outer diameter recess areas;
unfurling the rolled distal and proximal edges of the balloon away from one another covering the distal and proximal reduced outer diameter recess areas pooled with the biocompatible adhesive forming the respective distal and proximal bonding interface areas with the catheter shaft.

9. The balloon guide catheter of claim 1 or the method of claim 6, wherein each of the distal reduced outer diameter recess area and the proximal reduce outer diameter recess area is at least one radial groove extending 360° or less about a circumference of the exterior surface of the catheter shaft.

10. The balloon guide catheter of claim 9, wherein the at least one radial groove in each of the distal reduced outer diameter recess area and the proximal reduced outer diameter recess area extends 360° about a circumference of the exterior surface of the catheter shaft.

11. The balloon guide catheter of claim 9, wherein the proximal reduced outer diameter recess area is the at least one radial groove that extends less than 360° about a circumference of the exterior surface of the catheter shaft to avoid interference with the inflation lumen.

12. The method of claim 9, wherein the distal reduced outer diameter recess area is a first set of a plurality of longitudinally defined channels defined in the exterior surface of the catheter shaft separated in an axial direction from so as to be distinct from the proximal reduced outer diameter recess area having a second set of a plurality of longitudinally defined channels defined in the exterior surface of the catheter shaft.

13. The method of claim 9, wherein the distal reduced outer diameter recess area is a first set of at least one radial row of wells defined in the exterior surface of the catheter shaft separated in an axial direction distinct from the proximal reduced outer diameter recess area having a second set of at least one radial row of wells defined in the exterior surface of the catheter shaft.

14. The balloon guide catheter or the method of claim 7, wherein the radially inward depth of each of the distal and proximal reduced outer diameter recess areas is equal to that of the outer layer exposing the supporting layer beneath.

15. The balloon guide catheter or the method of claim 7, wherein the supporting layer is a braid: (i) encircling the central lumen; and (ii) woven above and below the inflation lumen.
